**Europäisches Patentamt**

**European Patent Office**

**Office européen des brevets**

(19)

(11) Veröffentlichungsnummer: **0 093 794**
**B1**

(12) # EUROPÄISCHE PATENTSCHRIFT

(45) Veröffentlichungstag der Patentschrift:
**15.10.86**

(51) Int. Cl.⁴: **A 61 M 13/00,** A 61 M 15/08

(21) Anmeldenummer: **82105511.8**

(22) Anmeldetag: **23.06.82**

(54) **Sauerstoff-Insufflationsbrille.**

(30) Priorität: **24.04.82 DE 3215466**

(43) Veröffentlichungstag der Anmeldung:
**16.11.83 Patentblatt 83/46**

(45) Bekanntmachung des Hinweises auf die Patenterteilung:
**15.10.86 Patentblatt 86/42**

(84) Benannte Vertragsstaaten:
**AT BE CH FR GB IT LI SE**

(56) Entgegenhaltungen:
**US-A-2 135 800**
**US-A-2 168 705**
**US-A-3 209 755**
**US-A-4 156 426**

(73) Patentinhaber: **Drägerwerk Aktiengesellschaft,
Moislinger Allee 53- 55, D-2400 Lübeck 1 (DE)**

(72) Erfinder: **Koch, Jochim, Dr., D-2411 Hollenbek (DE)**
Erfinder: **Drews, Wolfgang, Dipl.- Ing.,
Schillerstrasse 1, D-2067 Reinfeld (DE)**

EP 0 093 794 B1

## Beschreibung

Die Erfindung betrifft eine Sauerstoff-Insufflationsbrille nach dem Oberbegriff des Anspruchs 1.

In der Sauerstoff-Therapie wird dem Patienten ein Gemisch aus Sauerstoff und Luft zugeführt. Als eine für den Patienten sehr angenehme Methode hat sich dabei die nasale Insufflation erwiesen. Die Sauerstoffzufuhr erfolgt dabei durch einen Nasenkatheter, eine Kanüle oder durch eine Insufflationsbrille.

Eine bekannte Nasenkanüle zur Verabreichung von Sauerstoff an Patienten besteht aus einem oben abgeflachten Körper aus biegsamem Werkstoff, aus dessen oberer Fläche im Abstand voneinander zwei hohle, rohrförmige Ansätze vorstehen. Die Höhlungen beider Ansätze stehen in Verbindung mit einer den abgeflachten Körper längs durchsetzenden Gasführung. An die Gasführung ist an jeder Seite ein biegsamer Schlauch angeschlossen. Beide Schläuche sind mit einer gemeinsamen Sauerstoffquelle verbunden. Zur Benutzung werden die rohrförmigen Ansätze in die Nasenlöcher eingeführt, daß die obere Fläche des abgeflachten Körpers der Unterseite der Nase anliegt und die Nasenlöcher überspannt. Die biegsamen Schläuche werden beiderseits des Kopfes hinter den Ohren herumgeführt und unter dem Kinn lösbar zusammengefaßt, um einen zuverlässigen Sitz zu bewirken. Für die Langzeitanwendung ist dabei lästig, daß durch den Verschluß beider Nasenlöcher eine Ausatmung nur durch den Mund möglich ist. Zugleich wird der Klang der Sprache stark beeinflußt. Die in die Nasenlöcher eingeführten rohrförmigen Ansätze sind unangenehm. Die Führung der Schläuche über die besonders beweglichen Teile des Gesichtes stört bei der Sprache und wirkt entstellend. (DE-C- 20 55 506)

Eine bekannte Sauerstoffbrille für die Sauerstoff-Insufflation besitzt ein brillenartiges Gestell, das über die Nasenwurzel, die Nasenwände und unter den Augen verläuft und mit je einem Bügel hinter den Ohren befestigt wird. Das Gestell besteht aus einem Rohr und ist an einer Seite mit einer Tülle zur Sauerstoffeinspeisung versehen. Unterhalb jeden Auges ist je ein Nasenrohr angeschlossen, das gekrümmt bis vor die Nasenlöcher verläuft und am Ende einen kurzen Gummischlauch trägt, der je in das Nasenloch eingeführt wird. Für Langzeitanwendung wirkt die Sauerstoffbrille entstellend und ist beim Tragen einer Sehbrille hinderlich. Die in die Nasenlöcher eingeführten Schläuche sind unangenehm. Ein Verschluß der Nase ist nicht gegeben und dadurch wegen der Ausatmung nur eine mäßige Sauerstoffanreicherung erzielbar. (Prospekt 618, Aug. 1964, Drägerwerk)

Die Aufgabe der Erfindung ist eine Sauerstoff-Insufflationsbrille, die die obigen Nachteile nicht besitzt, sondern für den Patienten durch eine höhere Sauerstoffanreicherung in den Atemwegen die Atmung erleichtert, durch ihre Ausbildung eine flexible Anpassung an die Kopfform ermöglicht und das Tragen einer Sehbrille nicht behindert.

Diese Aufgabe wird ausgehend von einer Sauerstoff-Insufflationsbrille der im Oberbegriff des Anspruchs 1 gennanten Art erfindungsgemäß dadurch gelöst, daß das Gestell aus einem Stirnteil mit einem flexiblen Nasenanschluß, der in nur einem hohlen Nasenstopfen endet, und den in je einem Aufhänger mit einer innenliegenden spiralförmig gebogenen Feder endenden, über flexible Zwischenstücke am Stirnteil angeschlossenen Brillenbügeln besteht.

Die Vorteile dieser Sauerstoff-Insufflationsbrille ergeben sich aus der sehr guten Anpassungsmöglichkeit an alle Gesichtsformen und dem damit sehr bequemen Tragen, vor allem aus der Einblasung des Sauerstoffes durch nur ein im übrigen verschlossenes Nasenloch. Aus dem anderen Nasenloch kann der Patient ungestört ausatmen, während durch das weitere gleichzeitige Einblasen in einen Teil der Atemwege dann sofort wieder Sauerstoff zum Einatmen zur Verfügung steht und eine hohe Sauerstoffkonzentration in den Atemwegen sichert. Die günstige Form des Gestelles folgt der natürlichen Horizontal- und Vertikalteilung des Gesichts und verläuft von der Stirn zur Nasenwurzel. Der Sauerstoffversorgungsschlauch kann unauffällig hinter dem Ohr verlegt werden. Eine Entstellung des Gesichtes wird vermieden. Die hohe Lage des Stirnteils und der Abstand des Nasenanschlusses von der Nasenwurzel ermöglichen das gleichzeitige Tragen einer Sehbrille.

Um den Zufluß des Sauerstoffes kontrollieren zu können, ist der Sauerstoffversorgungsschlauch in weiterer Ausbildung der Erfindung mit einer Flowkontrolleinrichtung, die mittels einer Kammer einfach an der Kleidung getragen werden kann, versehen. Gleichzeitig werden dadurch Zugkräfte, die z.B. durch Hängenbleiben mit dem Sauerstoffversorgungsschlauch entstehen, abgefangen und nicht auf die empfindlichen Körperteile, wie Nase und Ohr, übertragen.

Weitere Ausbildungen gemäß den abhängigen Ansprüchen 3 bis 5 geben eine besonders günstige Auswahl von zweckmäßigen Materialien an.

Die insgesamt mit der Erfindung erzielten Vorteile gestatten dem Patienten, ohne Behinderung und Entstellung des Gesichtes mit dem für die Atmung notwendigen Sauerstoff versorgt zu werden.

Ein Ausführungsbeispiel der Erfindung ist in der Zeichnung dargestellt und wird im folgenden beschrieben. Es zeigen

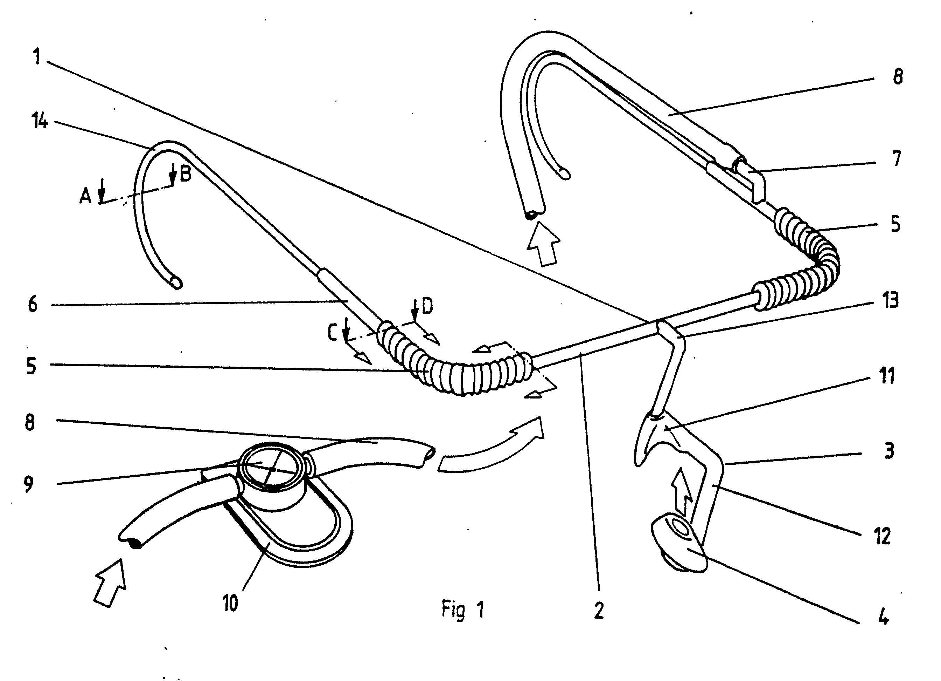

Fig. 1 die Gesamtansicht der Sauerstoff-Insufflationsbrille,

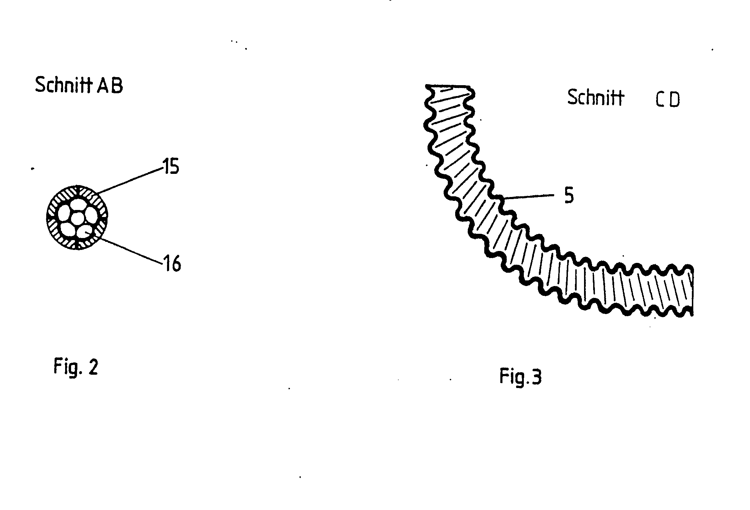

Fig. 2 einen Querschnitt A-B eines Aufhängers,

Fig. 3 einen Längsschnit C-D eines Zwischenstückes.

Die Insufflationsbrille 1 ist ein Gestell, das sich aus einem Stirnteil 2 mit einem flexiblen Nasenanschluß 3, der in einem hohlen Nasenstopfen 4 endet, und seitlich über flexible Zwischenstücke 5 angeschlossenen Brillenbügeln 6 zusammensetzt. Ein Brillenbügel 6 besitzt einen parallel oberhalb des Brillenbügels 6 verlaufenden Stutzen 7 für den Anschluß eines Sauerstoffversorgungsschlauches 8. Eine Flowkontrolleinrichtung 9, die mittels einer Klammer 10 an der Kleidung getragen werden kann, ermöglicht die Überwachung des Sauerstoffzuflusses.

Der flexible Nasenanschluß 3 ist ein Spritzteil aus einem bekannten Kunststoff. Er bildet eine Nasenauflage 11, an die sich einseitig eine hohle Verbindung 12 mit dem für das Nasenloch passenden Nasenstopfen 4 anschließt. An der Nasenauflage 11 ist der Nasenanschluß 3 mit dem vertikalen Schenkel eines im Winkel vorspringenden Rohrstutzens 13 des Stirnteils 2 verbunden. Die flexiblen Zwischenstücke 5 bestehen aus Wellrohr.

Die Brillenbügel 6 enden in einem Aufhänger 14 aus wendelförmig gewickelten Flachdrähten 15, der durch eine innenliegende, aus mehreren Federdrähten bestehende Feder 16 zur Spirale gebogen ist.

Durch die flexiblen Zwischenstücke 5, die Aufhänger 14 und den flexiblen Nasenanschluß 3 paßt sich die Insufflationsbrille 1 beim Aufsetzen federnd den gegebenen Kopfformen an. Diese sind bestimmt durch die Breite des Kopfes, den Abstand Nase/Ohr und die Nasenlänge. Das hochliegende Stirnteil 2 verläuft oberhalb der üblichen Sehbrillen-Fassungen, und unterhalb des gewinkelten Rohrstutzens 13 ist genügend Raum auch für hochliegende Mittelstege von Sehbrillen, so daß das Tragen der Insufflationsbrille 1 zusätzlich zu einer Sehbrille möglich ist.

**Patentansprüche**

1. Sauerstoff-Insufflationsbrille (1) aus einem Gestell aus Rohrmaterialien mit seitlichen Brillenbügeln (6), von denen einer einen Stutzen (7) für den Anschluß eines Sauerstoffversorgungsschlauches (8) besitzt, dadurch gekennzeichnet, daß das Gestell aus einem Stirnteil (2) mit einem flexiblen Nasenanschluß (3), der in nur einem hohlen Nasenstopfen (4) endet, und den in je einem Aufhänger (14) mit einer innenliegenden spiralförmig gebogenen Feder (16) endenden, über flexible Zwischenstücke (5) am Stirnteil (2) angeschlossenen Brillenbügeln (6) besteht.

2. Sauerstoff-Insufflationsbrille nach Anspruch 1, dadurch gekennzeichnet, daß der Sauerstoffversorgungsschlauch (8) zwischengeschaltet eine mit einer Klammer (10) versehene Flowkontrolleinrichtung (9) enthält.

3. Sauerstoff-Insufflationsbrille nach Anspruch 1 und 2, dadurch gekennzeichnet, daß die flexiblen Zwischenstücke (5) aus Wellrohr hergestellt sind.

4. Sauerstoff-Insufflationsbrille nach Anspruch 1 bis 3, dadurch gekennzeichnet, daß die Aufhänger (14) aus wendelförmig gewickelten Flachdrähten (15) und die innenliegende Feder (16) aus mehreren Federdrähten bestehen.

5. Sauerstoff-Insufflationsbrille nach Anspruch 1 bis 4, dadurch gekennzeichnet, daß der flexible Nasenanschluß (3) ein Kunststoff-Spritzteil ist.

**Claims**

1. Oxygen insufflation spectacles (1) consisting of a tubular frame with lateral spectacle arms (6), one of which has a connecting piece (7) for the connection of an oxygen supply tube (8), characterised in that the frame consists of a front piece (2) with a flexible nose connection (3), which ends in just one hollow nose plug (4), and the spectacle arms (6) which end, in each case, in a hook (14) with a bent spiral spring (16) lying therein and which are connected to the front piece (2) by flexible intermediate pieces (5).

2. Oxygen insufflation spectacles according to claim 1, characterised in that the oxygen supply tube (8) contains, interposed therein, a flow control device (9) which is provided with a clamp (10).

3. Oxygen insufflation spectacles according to claims 1 and 2, characterised in that the flexible intermediate pieces (5) are made of corrugated tubing.

4. Oxygen insufflation spectacles according to claims 1 to 3, characterised in that the hooks (14) consist of helically coiled flat wires (15) and the spring (16) lying therein consists of several spring wires.

5. Oxygen insufflation spectacles according to claims 1 to 4, characterised in that the flexible nose connection (3) is made of a moulded plastics material.

**Revendications**

1. Lunettes (1) à insufflation d'oxygène, constituées d'une monture réalisée en matériaux tubulaires, avec des branches latérales (6) de lunettes dont l'une possède une tubulaire (7) pour le raccordement d'un flexible (8) d'alimentation en oxygène, caractérisées en ce que la monture est constituée d'une partie frontale (2) munie d'un raccord nasal flexible (3) qui se termine.par un seule embout nasal creux (4), et des branches de lunettes (6) qui sont assemblées à la partie frontal (2) par l'intermédiaire d'éléments intermédiaire (5) flexibles, et qui se terminent chacune par une attache (14) à l'intérieur de laquelle se trouve un ressort (16) coudé en hélice.

2. Lunettes à insufflation d'oxygène selon la revendication 1, caractérisées en ce qu'un appareil de contrôle de débit (9) muni d'une agrafe (10) est interposé dans le flexible d'alimentation en oxygène (8).

3. Lunettes à insufflation d'oxygène selon la revendication 1 ou 2, caractérisées en ce que les éléments intermédiaires flexibles (5) sont réalisés en tube ondulé.

4. Lunettes à insufflation d'oxygène selon une des revendications 1 à 3, caractérisées en ce que les attaches (14) sont constituées de fils métalliques plats (15) enroulés en hélice, et que les ressorts (16) situés à l'intérieur sont constitués de plusieurs fils d'acier à ressort.

5. Lunettes à insufflation d'oxygène selon une des revendications 1 à 4, caractérisées en ce que le raccord nasal flexible (3) est une pièce en matière plastique moulée par injection.

Fig 1

0 093 794

Schnitt AB

Fig. 2

Schnitt CD

Fig. 3

0 093 794